# EUROPEAN PATENT APPLICATION

(11) **EP 4 197 556 A1**
(43) Date of publication of application: **21.06.2023**
(21) Application number: 21216014.7
(22) Date of filing: 20.12.2021
(51) Int. Cl.: A61K 41/00, A61K 49/00, A61B 17/00, A61L 31/06, A61N 5/06

(54) **COMPOSITION FOR LASER TISSUE SOLDERING**

(71) Applicant: ETH Zurich, 8092 Zurich (CH)
(72) Inventor: HERRMANN, Inge, 8052 Zürich (CH); CIPOLATO, Oscar, 8052 Zürich (CH)

(57) **Abstract**

The present invention relates to a composition comprising a temperature sensitive solder and at least one type of nanoparticles, characterized in that the first type of nanoparticles is a fluorescent nanothermometer, wherein said fluorescent nanothermometer exhibits an excitation maximum and temperature dependent emission spectrum each in the range of between 650 and 1350 nm. The composition according to the present invention can be used for laser tissue soldering.

## Description

The present invention relates to a composition for laser tissue soldering.

Almost every surgical intervention requires some type of joining of adjacent sections of tissue. Technologies such as sutures, staples, clips, and adhesives have been used for many years to close surgical incisions and wounds.

When suturing, the surgeon passes the needle through the tissues close to their edges, pulling them together and thereby relying on tissue and suture strength to hold them tight. Thus, suturing is a delicate, demanding, and time-consuming procedure which requires technical skills. Sutured sites are at an increased risk for leaks, infections, and scarring.

Stapling is appropriate in some cases, but it also relies on the original tissue strength and may be limited by the shape and size of the specific stapler since a poorly adapted stapler may result in bonding failure. Adhesives also have their limitations since they may exhibit insufficient tensile strength and may be toxic.

Methods of wound closure have been continuously evolved, and newer and better techniques have been developed. One of these methods is laser tissue bonding. Broadly, laser tissue bonding is divided into three types, namely laser tissue welding, laser tissue soldering and dye-enhanced laser tissue soldering.

Laser tissue soldering is a technique that is able to form strong bonds. Thermal control is of utmost importance in laser tissue soldering. Temperatures below the target temperature do not allow the formation of strong bonds, while temperatures above the target temperature will damage the surrounding tissues and hinder the healing process.

Infrared thermal cameras and sensors have been used for thermal sensing (for example Gerasimenko et al. "Laser System with Adaptive Thermal Stabilization for Welding of Biological Tissues" in Biomed Eng 49, pages 344-348 (2016)). However, the radiation used for temperature sensing is not within the biological window. Therefore, it is only possible to get information of the surface of the tissue. Non-superficial wounds cannot be controlled. Moreover, optics for their wavelength are expensive.

Dong et al. "Terahertz three-dimensional monitoring of nanoparticle-assisted laser tissue soldering", Vol. 11, Issue 4, pages 2254-2267 (2020) disclose the investigation of the thermal damage during the soldering process by terahertz radiation. However, this method is based on the water content in the tissue and is not able to give information about the temperature during the soldering process.

Khosroshahi et al. "Application of Albumin Protein and Indocyanine Green Chromophore for Tissue Soldering by Using an IR Diode Laser: Ex Vivo and In Vivo Studies" Photomedicine and Laser Surgery 28(6), pages 723-33 (2010) disclose the colour change of indocyanine green during soldering process as indicator for the end of the soldering process. However, said method is not very precise.

The problem of the present invention is therefore to provide a composition for laser tissue soldering that allows precise temperature control during the soldering process.

The problem is solved by the composition according to claim 1. Further preferred embodiments are subject of the dependent claims.

The composition according to the present invention comprises a temperature sensitive solder and at least one type of nanoparticles. Said first type of nanoparticles is a fluorescent nanothermometer exhibiting an excitation maximum and a temperature dependent emission spectrum each in the range of between 650 and 1350 nm.

The expression "temperature sensitive solder" is known in the art. Temperature sensitive solders are for example protein solders or chitosan solders. Protein solders are preferably selected from the group consisting of albumin, fibrinogen, collagen and gelatin, which denature upon exposure to localized heating and which are further capable of cross-linking to each other and to the adjacent tissue to which the composition of the present invention is contacted.

Within the context of the present invention, the expression "fluorescent nanothermometer" stands for nanoparticles and structures which exhibit an excitation maximum and temperature dependent emission spectrum each in the range of between 650 and 1350 nm. Said nanothermometers are capable of fluorescence, i.e. they absorb electromagnetic radiation (excitation process) followed by the spontaneous emission of electromagnetic radiation (emission process). As both the excitation maximum and the emission maximum are in the range of between 650 and 1350 nm, fluorescent nanothermometers according to the present invention work within the biological window, i.e. the biological cells are not negatively impacted by the electromagnetic radiation. When applying the composition according to the invention, the ratio of the excitation maximum and the emission maximum of the fluorescent nanothermometer is used to calculate the temperature of the temperature sensitive solder. Band-shape fluorescent nanothermometry is a preferred method as said method is robust and flexible. However, other methods such as intensity, spectral, polarization, bandwidth and lifetime luminescence nanothermometry are possible. Said methods are known to the skilled person. The fluorescent nanothermometers have a particle size that allows an easy dispersion. Typically, they have a mean crystal size of 10 to 200 nm (measured by x-ray diffraction or electron microscopy). The temperature sensitivity of the fluorescent nanothermometers can be optimized for example by doping at particular percentages in a particular host or by coupling more dyes.

The composition according to the present invention allows a sensitive and accurate measurement of the temperature at the interface between temperature sensitive solder and tissue in real time. Since the composition according to the present invention allows to work in the biological window, it can provide information about the temperature even when applied in wounds at tens of millimetres below the surface. Thus, it can be used for both, superficial and non-superficial wounds and can therefore greatly improve the soldering process.

The composition according to the present invention allows a soldering process resulting in a tissue bond with sufficient surgical tensile strength but only minimal immediate and long-term denaturation of the surrounding tissue.

In addition, the composition according to the present invention requires a less expensive setup equipment than systems used in the prior art.

In a preferred embodiment, the composition according to the present invention can additionally comprise a photothermal agent, wherein said photothermal agent is preferably selected from the group consisting of plasmonic nanoparticles and photothermal dyes.

Within the context of the present invention the expression "photothermal agent" stands for agents that can convert laser light into heat in the biological window, i.e., in a wavelength range of 650 to 1350 nm. Due to their high absorption coefficient and photothermal energy conversion efficiency, both, plasmonic nanoparticles and photothermal dyes can efficiently convert light to heat in the biological window. Thus, the photothermal agent has a heating function, while the fluorescent nanothermometer has a thermal sensing function. Fluorescent nanothermometers do not typically provide plasmonic properties.

Within the context of the present invention near IR absorbing dyes of the present disclosure can absorb optical radiation within the near IR spectrum and convert the absorbed radiation to thermal energy.

A laser is a device that emits light through a process of optical amplification based on the stimulated emission of electromagnetic radiation. A laser differs from other sources of light in that it emits light which is coherent. If the electromagnetic radiation of a laser source has a wavelength range of 650 to 1350 nm, the photothermal agents can selectively absorb incident light in the biological window and convert it to localized heat.

The presence of the two types of particles, namely the fluorescent nanothermometers and the photothermal agents, has a synergistic effect. The addition of the fluorescent nanothermometers increases the scattering coefficient of the material which in turn increases its extinction coefficient. Thus, the increased scattering favours the absorption of laser light by the photothermal agents and consequently its heat production. This allows to reduce the amount of photothermal agents needed in the composition of the present invention.

Preferably, the fluorescent nanothermometers are selected from the group consisting of quantum dots, nanodiamonds, gold nanoclusters, hosted rare earth ions, upconverting nanoparticles, transition metal ions, luminescent organic dyes, luminescent organic polymers and luminescent gels.

Within the context of the present invention the expression "quantum dots" refers to particles which have a three-dimensional size in nanoscale (less than 100 nm), and are composed of finite number of atoms. Preferably, said quantum dots are selected from the group consisting of carbon dots, CdSe, CdS, Ag₂S and PbS, preferably CdSe, CdS, Ag₂S and PbS.

Within the context of the present invention the expression "upconverting nanoparticles" refers to nanoparticles that when excited with light emit light that has a wavelength that is shorter than the excitation wavelength. Typically, the comprise a host crystal which is doped with one or several types of lanthanide ions. Typical host materials are metal fluorides (NaYF₄), oxides (Y₂O₃), phosphates (YPO₄), and vanadates (GdVO₄). The lanthanide ions are preferably selected from the group consisting of Er³⁺, Yb³⁺, Ho³⁺, Gd³⁺, Tm³⁺ or combinations thereof.

Within the context of the present invention the expression "hosted rare earth ions" refers to host lattice materials containing dopant rare earth ions. Examples for such hosts are LaF₃, Ba₃(PO₄)₂ and BiVO₄. The rare earth ions are preferably selected from the group consisting of Eu³⁺, Nd³⁺, Mn³⁺, Er³⁺ and Dy³⁺.

Within the context of the present invention the expression "nanodiamonds" refers to truncated semioctahedral carbonaceous architecture ranging from 5 to 50 nm in diameter.

Within the context of the present invention "gold nanoclusters" refers particles with sizes of less than 2 nm, comprising up to hundreds of gold atoms. Gold nanoclusters may optionally be protected by ligands such as sulfonic thiolate ligands or by thiolated peptides. Examples of gold nanoclusters are Au₈, Au₁₃, Au₂₀, Au₂₄, Au₃₂ and Au₃₉.

Examples for fluorescent organic dyes having nanothermometer properties are (E)-2-chloro-N-(4-(5,5-difluoro-3-(2-hydroxy-5-(trifluoromethoxy)styryl)-1-methyl-5H-4λ4, 5λ4-dipyrrolo[1,2-c:2',1'-f][1,3,2]diazaborinin-10-yl)phenyl)acetamide (ER thermo Yellow), Rhodamine 800 and *N*,*N*-dibutyl-6-imino-9-(3-piperidin-1-ylphenyl)xanthen-3-amine (Mito thermo yellow).

Examples for luminescent organic polymers having nanothermometer properties are thermosensitive polymer such as NIPAM (poly(N-isopropylacrylamide), OEGMA (oligo(ethylene glycol) methyl ether methacrylate), NNPAM (N-n-propylacrylamide), PDA (perfluoralkoxy polymere), PMMA (poly(methyl-2-methylpropenoate)) which are linked with a dye (e.g. DBD-AA (N-(2-{[7-(N,N-dimethylaminosulfonyl)-2,1,3-benzoxadiazol-4-yl]-(methyl)amino}ethyl)-N-methylacrylamide), ICG (indocyanine green), AMC (7-amino-4-methyl-cumarine), NBD (4-chlor-7-nitro-benzofurazane).

Examples for luminescent gels are cationic nanogels of N-isopropylacrylamide (NIPAM), including NIPAM-based cationic fluorescent nanogel thermometers.

The expression "doped with a specific cation" means the replacement of a host cation with said specific cation. Typically, 0.05 to 5 at%, preferably 0.05 to 2 at% of the host cation is replaced with the specific cation. The unit at% is defined as the atomic fraction of the total metal ion concentration. These nanoparticles provide a high chemical-, thermal- and photo-stability. Furthermore, they exhibit no blinking. The absence of blinking significantly improves signal-to-noise ratio. In addition, they do not exhibit bleaching, so that the signal does not disappear over time.

Preferred fluorescent nanothermometers are selected from the group consisting of neodymium-doped BiVO₄, manganese (V) doped Ba₃(PO₄)₂, LiNdP₄O₁₂, neodymium-doped CaF₂, and neodymium-doped LaF₃. Said fluorescent nanothermometers, and in particular neodymium-doped BiVO₄, exhibit excellent reproducibility while showing negligible dependence on experimental parameters and surroundings, which is of utmost importance for biological tissue application.

The composition according to the present invention can comprise plasmonic nanoparticles having a broad absorption range or plasmonic nanoparticles with steep absorption peeks. In one embodiment, the composition of the present invention comprises a mixture of different plasmonic nanoparticles. The plasmonic nanoparticles can be in the shape of spheres, core-shell structures, Janus-particle structure, rods, stars, triangles or cubes.

Preferably, the plasmonic nanoparticles are selected from the group consisting of plasmonic metal nanoparticles, plasmonic carbon nanotubes, plasmonic organic nanoparticles, plasmonic conductive oxides and plasmonic conductive transition metal nitrides, hybrids, silica coated nanoparticles or mixtures thereof, preferably plasmonic metal nanoparticles, wherein the metal is selected from the group consisting of Au, Al, Ag, Cu, Pt and Pd, most preferably Au. Gold nanostructures include gold nanoparticles, gold nanorods, gold nanospheres and gold nanostars. Typically, the plasmonic nanoparticles are present in a concentration of about 0.005 to 1 mg/ml.

In particular, conductive transition metal nitrides selected from the group consisting of TiN, ZrN, TaN and HfN are very efficient light-to-heat converter. They have a broad absorption range and can be produced easily. Furthermore, TiN is inexpensive and already being used as a coating for biomedical implants and many other biological applications, which allows a safe application in humans.

Conductive oxides are preferably selected from the group consisting of RuO₂, Al-doped ZnO, Ga-doped ZnO, HfO₂, I-doped SnO₂ and F-doped SnO₂.

A gold nanorod is defined as nanometer-sized gold particles that are rod shaped. Typically, it has a diameter ranging from a few nanometers to hundreds nanometers with aspect ratios (length/diameter) larger than 1 but typically smaller than 6.5. Such gold nanorods have a steep absorption peak at about 1064 nm. Gold nanorods have the advantage that their steep absorption can be tuned by changing their aspect ratio. Furthermore, depending on their aspect ratio, they are able to absorb selectively at the required wavelength, including the ones in the range 900-1300 nm.

The composition according to the present invention can also comprise photothermal dyes. Said photothermal dyes are preferably are near-infrared absorbing dyes, preferably selected from the group consisting of indocyanine green, brilliant blue green, bromophenol blue, 4-[2-[2-chlor-3-[(2,6-diphenyl-4*H*-thiopyran-4-yliden)-ethyliden]-1-cyclohexen-1-yl]ethenyl]-2,6-diphenylthiopyrylium-tetrafluoroborat (IR-1061), diimminium dye and cyanine dyes.

In one embodiment of the present invention, the excitation maximum of the fluorescent nanothermometer differs from the absorption maximum of the plasmonic nanoparticle by at least 200 nm. This allows to control the laser soldering process by two lasers. One laser is tuned at the wavelength of the fluorescent nanothermometer, and the second laser is tuned at the wavelength of the plasmonic nanoparticles. Thus, one laser is for thermal sensing, while the other is for heating. This allows to interrupt the heating process but to continue the temperature control.

In another embodiment of the present invention, only one laser is used during the soldering process. Said laser is tuned for nanothermometer excitation and photothermal agent absorption which allows a cost-effective equipment.

Best results can be obtained with a composition according to the present invention comprising neodymium-doped BiVO₄ as fluorescent nanothermometers and TiN and/or gold nanorods as plasmonic nanoparticles.

Preferably, the temperature sensitive solder contained in the composition according to the present invention comprises of serum albumin, chitosan, collagen, fibrinogen and gelatin or a mixture thereof, preferably bovine serum albumin (BSA), human serum albumin (HAS) and gelatin or a mixture thereof, most preferably a mixture of bovine serum albumin (BSA) and gelatin or human serum albumin (HAS) and gelatin. The presence of albumin improves the bonding strength of the bond. Gelatin has the advantage that it is jelly-like at room temperature and liquifies at temperatures above 35°C. The presence of gelatin allows for example the formation of strips and patches of the desired geometry as well as the formation of strong bonds. Chitosan provides very good bond strengths and/or burst pressures. In addition, chitosan is hemostatic, mucoadherent and biodegradable which all makes it well suited for use in tissue repair applications. Chitosan is preferably present in combination with serum albumin.

In one embodiment of the present invention, the temperature sentitive solder additionally comprises a biodegradable polymer selected from the group consisting of poly(L-lactic acid) (PLA),poly(glycolic acid), poly(L-lactic-co-glycolic acid) (PGA), poly([epsilon]-caprolactone) (PLGA) and polyortho esters, polyanhydrides or combinations thereof. Said biodegradable polymers work as scaffold and allow the formation of strips of the desired geometry as well as the formation of a strong bond.

In one embodiment of the present invention the fluorescent nanothermometers and the plasmonic nanoparticles are present in a ratio of 1:1 to 5000:1 % by weight. Due to increased scattering coefficient given by the fluorescent nanothermometers, the concentration of the plasmonic nanoparticles can be significantly reduced.

Preferably, the composition comprises 0.01 to 100 mg/ml, preferably 0.1 to 50 mg/ml, most preferably 1 to 10 mg/ml of the fluorescent nanothermometers. This concentration guarantees an optimal sensitivity, and in some applications, it can even eliminate the need for further calibrations after conducting the initial calibration.

In a further embodiment, the composition according to the present invention additionally comprises a therapeutic agent selected from the group consisting of antimicrobial agents, anti-inflammatory agents and angiogenic agents. Antimicrobial agents are antibiotics, antibacterials, antifungals, antivirals and antiprotozoans. Anti-inflammatory agents are compounds that reduce inflammation or swelling. Possible anti-inflammatory agents are in particular nonsteroidal anti-inflammatory drugs and/or radical scavengers (such as vitamins, metal oxides, including iron oxide, cerium oxide, manganese oxide and cobalt oxide). As part of the composition according to the present invention, the therapeutic agent is directly applied to the surgical site resulting in a rapid wound healing. Furthermore, a lower dose of said therapeutic agents is possible since no systemic administration is needed. These optionally added therapeutic elements can give rise to synergistic properties and enable photodynamic or photothermal therapy using the solder material.

Preferably, the composition according to the present invention is formulated as paste, gel, strip, patch or viscous fluid having a viscosity of at least 2'000 centipoises at 25°C and can be applied directly on the wound. A patch may be customized to desired size and shape by the surgeon and is particularly suitable for large wounds, whereas pastes, gels and strips are preferably used for small wounds.

In another embodiment, the composition of the present invention is in lyophilised form. The term "lyophilise" with regard to the composition according to the present invention is intended to refer to the freeze-drying of an aqueous solution of the composition. The term "lyophilisate" refers to the product of lyophilisation. According to one embodiment of the invention, the lyophilised composition further comprises a suitable buffering agent for maintaining a pH of about 5.5 to 7.5, preferably of about 6.0 to 7.0, and more preferably about 6.3 to 6.7 after reconstitution of the lyophilisate. The term "reconstitution" refers to immersion of the lyophilisate in an aqueous medium for achieving a hydrated form. A buffering agent of the composition according to the invention may be selected from a group comprising of disodium phosphate dihydrate, sodium dihydrogen phosphate dihydrate, trisodium citrate dihydrate or combination thereof. The lyophilised form of the composition according to the present invention results in an improved long-term stability. In addition, the lyophilised form of the composition according to the present invention can be stored at room temperature or in the fridge. Storage in the fridge is preferred.

One aspect of the present invention relates to the use of the composition according to the present invention in the treatment of biological tissue repair by laser soldering. The composition of the present invention can be used for any tissue repair in individuals, and in particular for anastomosis (vessels, tissues and nerves); repair surgeries for skull base, cerebrospinal fluid leak (e.g. endoscopic endonasal surgical repair), nerve, tendon, muscle, fascia, vessel, lungs, diagphram, and esophagus (e.g. iatrogenic esophageal perforation); surgeries (possibly also for repairs) in urologic, gynecologic, orofacial, aerodigestive, gastrointestinal, ophthalmological and cerebrospinal fields (e.g. urologic/gynecologic endoscopic pelvic repairs, repairs after fibroidectomies, uterine closure, dental replacement, skin closure, bladder surgery, laparoscopic abdominal surgical repairs) .

In a preferred embodiment of the present invention, the composition of the present invention is used in the treatment of biological tissue repair by laser soldering, wherein the tissue is selected from the group consisting of cornea, blood vessels, fascia, diaphragm, anastomosis of vessels and tissues, dura mater leaks, gastrointestinal tissues and nerves. Said tissues are complex and delicate and would be damaged by conventional suturing.

A further aspect of the present invention relates to the use of a composition according to the present invention in the treatment of wound of an individual, wherein the individual is selected from the group consisting of elderly, diabetics, immunosuppressed individuals and immobilized individuals. Said individuals develop wounds that are slow to heal, do not heal well, or even never heal. Sometimes, an infection might develop. An infection can spread to tissue and bone near the wound or more distant areas of the body. In some cases, and without emergency care, an infection can be life-threatening or may even be fatal. Due to the composition according to the present invention, it is possible to obtain a fast and strong bonding of the tissue. In addition, the inflammatory response and the post-surgical recovery period is significantly reduced.

The composition according to the present invention may be used as follows: first the composition is placed on the wound. Then, the composition is irradiated with a laser light having a wavelength of 650 nm to 1350 nm. The laser light activates the fluorescent nanothermometer, and if present, the plasmonic nanoparticles. The composition of the present invention heats up the underlying tissue, and thus creates a continuous bond. The result is a waterproof seal along the entire wound which prevents access to external pathogens and does not require suture removal.

The present invention also includes the use of a laser to facilitate wound healing. Lasers are known to be used in wound healing and can be used with the compositions comprising a temperature sensitive solder. While any suitable laser can be used, a near IR laser (e.g., IR-A), such as from about 650 nm to 1350 nm may be advantageously used. The laser can be, for example, a single-wavelength laser, tuned for nanothermometer excitation and plasmonic particle absorption. Advantageously, two lasers are simultaneously used, one for thermal sensing, the other for heating. A spectrometer or a NIR camera is preferably used to measure the fluorescence.The resulted data are needed to calculate the temperature of the solder.

### Figures

Figure 1 shows the temperature increase in the composition with different compositions (all containing BSA and gelatin as temperature sensitive solder) under laser light irradiation at 750 nm.
Figure 2 shows a scheme of the laser tissue soldering setup.
Figure 3 shows a calibration curve that relates temperature and spectra at different temperatures (through the Fluorescence Intensity Ratio FIR).
Figure 4 shows a temperature during laser tissue soldering measured using a thermal camera ("Reference temperature") and the fluorescent nanothermometers ("Computed temperature") (using Nd-doped BiVO₄ at 2.5 mg/ml and TiN at 0.02 mg/ml; BSA 0.250 g/ml and gelatin 0.065 g/ml solution dissolved in PBS (phosphate-buffered saline)).
Figure 5 shows a typical stress-strain graph of a laser soldered tissue (BSA 60%, TiN at 1%.).

### Examples

### Preparation of the composition

First, the fluorescent nanothermometers need to be prepared. Luminescent Nd-doped BiVO₄ particles were prepared by flame spray pyrolysis (FSP). Bismuth (III) nitrate pentahydrate (Bi(NO₃)₃*5H₂O) and stoichiometric amounts of vanadium (ammonium metavanadate, NH₄VO₃) were dissolved separately in a 2:1 volumetric mixture of 2-ethylhexanoic acid and acetic anhydride under magnetic stirring for two hours at 100 °C. The Nd³⁺ is added by dissolving neodymium nitrate hexahydrate (Nd(NO₃)3*6H₂O). The Nd³⁺ concentration was defined as atomic fraction (at%) of the total metal ion concentration resulting in the formula Bi_{0.99} Nd_{0.01}VO₄, which has been shown to lead to the highest luminescence intensity. The total metal concentration was kept constant at 0.4 M. All chemicals were supplied by Sigma-Aldrich. The resulting precursor solution was fed at a constant rate (8 ml/min) through a nozzle and dispersed by 3 l/min of oxygen resulting in a fine spray that was ignited and sustained by a surrounding premixed oxygen/methane (1.5/3.2 l/min) flamelet. The particles were collected on a glass microfibre filter (Whatman GF) with the aid of a vacuum pump (Busch Mink MM 1202 AV) . The collected particles were sieved (standard mesh size of 250) and annealed for 6 h at 600 °C (Carbolite Gero 30-3000 °C).

When the nanothermometers are ready, the fabrication of the composition can begin. Various final concentrations of BSA (Sigma-Aldrich, lyophilised powder, ≥ 96%), gelatin (Sigma-Aldrich, from Porcine Skin, type-A, strength 300), BiVO₄ and TiN (PlasmaChem, 20 nm) can be selected. In this case the final concentrations (w/v) are BSA 25%, Gelatin 6.5%, Nd-doped BiVO₄ 0.2%, and TiN 0.003%. The composition is made from four starting solutions. A solution of 50% BSA was prepared by dissolving BSA in PBS. The dissolution speed can be increased using a mixer. A solution of 26% gelatin was prepared by adding gelatin to PBS. The solution was mixed and heated in a heated shaker (LLG Labware, uniTHERMIX 1) at 400 rpm and 60 °C until the solution was completely dissolved (usually in less than 30 min). A 1% solution of Nd-doped BiVO₄ in water and a 0.1% solution of TiN were prepared and put in an ultrasonicator. Then, 0.5 ml of 26% gelatin were brought to 42 °C and 1.224 mL of 50% BSA were added to it. The mixture was vortexed for about 10 seconds and put in the shaker at 42 °C. Then, 0.349 ml of Nd-doped BiVO₄ solution were added and the obtained solution quickly vortexed for a few seconds. The process was repeated using instead of the BiVO₄ solution 52.4 µl of TiN solution. Before the solution cooled down, it was poured into the desired mould, making sure that no bubbles were present. The composition was put in the fridge until gelation.

The presented protocol can be used to prepare compositions with other concentrations.

For example, compositions comprising only fluorescent nanothermometers can be created by using distilled water instead of solutions comprising plasmonic nanoparticles.

### Results and discussion

### Heating of the composition

The temperature that can be reached by shining a laser on the composition depends on the optical properties of the solder, mainly given by the nanothermometers and in particular by the plasmonic nanoparticles that are added. Whether a certain mix of nanoparticles can be used for laser tissue soldering depends on its ability to convert laser light into heat efficiently.

Three different compositions were used in order to investigate the temperature reached during soldering: two with either only TiN or only Nd-doped BiVO₄, and one with both types of nanoparticles (same concentrations described in the Materials section). A composition of the aforementioned formulation with a thickness of 0.5 mm was used. A 750 nm multimode CW laser (Laser Century, RLM750TA) is coupled to a fibre and used with a collimator. Laser power of 0.886 ± 0.001 W was used. The laser was shined diagonally, leading to an elliptical beam spot size of 1.5×1.0 cm (area of 2.36 cm²). The temperature is measured using a thermal camera (Fluke Ti110).

### Results and discussion

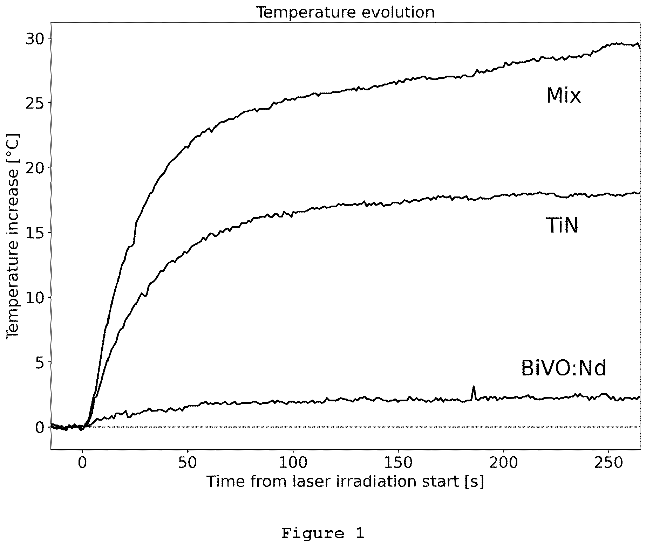

The measurements of temperature increase for the various compositions are shown in Fig. 1. The increase in temperature from the sample with only Nd-doped BiVO₄ is very low, while the temperature increase in the samples that contain TiN is much more pronounced. The increase in temperature for the compositions with the mix of nanoparticles is higher than the one for the compositions with only one type of nanoparticle. Not only that, but it is also higher than the sum of the temperature increases of the two other compositions. This can be attributed to the increased scattering coefficient given by Nd-doped BiVO₄ that allows more light to be absorbed by TiN.

### Temperature measurements through fluorescent nanothermometers

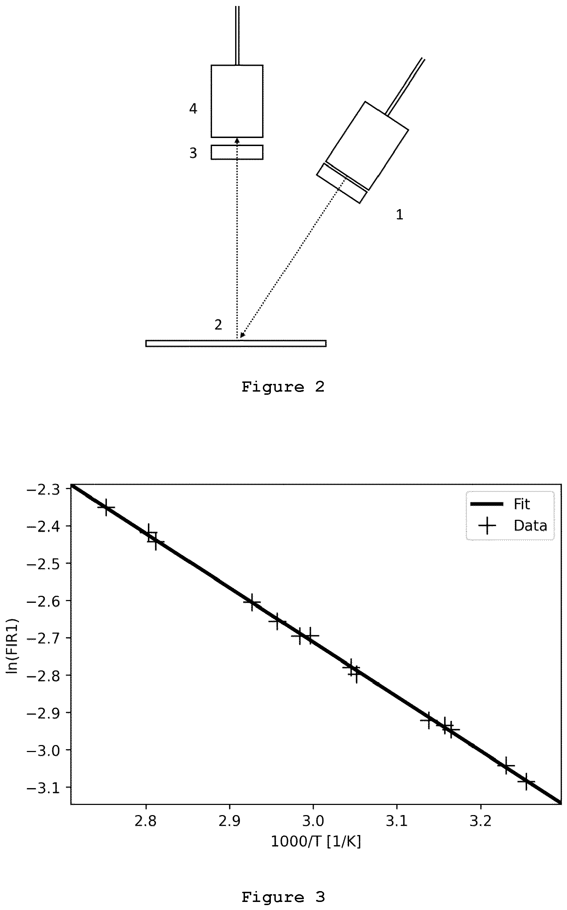

In order to retrieve temperature information from the fluorescent nanothermometers, they need to be calibrated. The nanothermometer powder was placed on a heating plate. A thermal camera is used for reference temperature measurement. The fluorescent spectrum is acquired by a spectrometer (Ocean Insight, STS-NIR). The signal is collected by a collimator and goes to the spectrometer through an optical fibre. A bandpass filter (Thorlabs, FB750-10) is placed in front of the laser source and two longpass filters at 780 nm (Edmund Optics) are placed in front of the spectrometer. The same setup (without the heating plate in the bottom) is also used for the soldering process. A scheme of the setup is shown in Fig. 2. A laser beam (1) comes out of a fibre collimator and bandpass filter and hits the sample (2). The light emitted by the sample passes through a longpass filter (3) and is finally focussed by a collimator (4) into a fibre connected to a spectrometer.

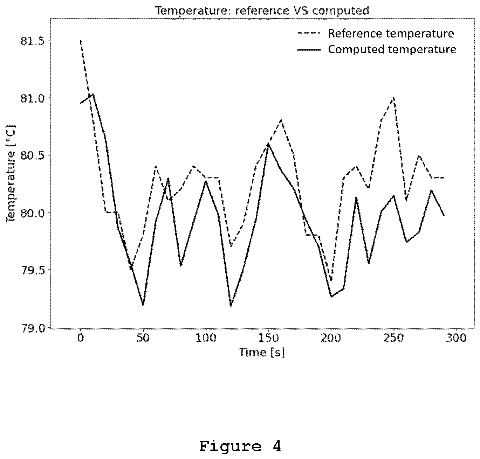

A calibration curve (see Fig. 3) is obtained from the spectra at different temperatures, using the same method explained by Gschwend et al. (P. M. Gschwend, F. H. L. Starsich, R. C. Keitel, and S. E. Pratsinis, "Nd3+-Doped BiVO4 luminescent nanothermometers of high sensitivity," Chem. Commun., vol. 55, no. 50, pp. 7147-7150, 2019). In order to validate the effectiveness of the temperature measurements, the temperature during laser tissue soldering has been measured using both a thermal camera and the nanothermometers (see Fig. 4), using 10 seconds as integration time for the spectrometer.

### Tensile strength

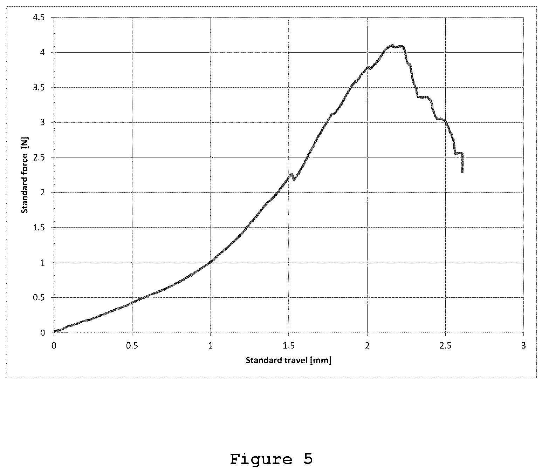

Preliminary data on the soldering strength have been taken using a solder that contained only TiN. A universal testing machine (Zwick) was used to measure the tensile strength of the samples. Pig small intestine was retrieved from a local slaughterhouse, cleaned with distilled water and frozen. Samples of around 5 × 4 cm² were cut. Another side-to-side cut was placed in the middle of the sample, parallel to the short side. The composition was placed on this cut and the soldering process was carried out: laser light with a circular spot size of 0.8 cm² and intensity of 2.5 W/cm² was shined on the solder and the sample moved when a temperature of 80 °C was measured with a thermal camera. The tensile properties of the samples were measured soon after. Tensile strengths of about 5 N were measured (a typical stress-strain graph is shown in Fig. 5).

## Claims

1. Composition comprising a temperature sensitive solder and at least one type of nanoparticles, **characterized in that** the first type of nanoparticles is a fluorescent nanothermometer, wherein said fluorescent nanothermometer exhibits an excitation maximum and a temperature dependent emission spectrum each in the range of between 650 and 1350 nm.

2. Composition according to claim 1, **characterized in that** the composition additionally comprises a photothermal agent, wherein the photothermal agent is preferably selected from the group of plasmonic nanoparticles and photothermal dyes.

3. Composition according to claims 1 to 2, **characterized in that** the fluorescent nanothermometers are selected from the group consisting of quantum dots, nanodiamonds, gold nanoclusters, hosted rare earth ions, upconverting nanoparticles, transition metal ions, luminescent organic dyes, luminescent organic polymers and luminescent gels, preferably hosted rare earth ions selected from the group consisting of neodymium-doped BiVO₄, manganese (V) doped Ba₃(PO₄)₂, LiNdP₄O₁₂, neodymium-doped CaF₂, and neodymium-doped LaF₃, and most preferably neodymium-doped BiVO₄.

4. Composition according to any one of claims 1 to 3, **characterized in that** the plasmonic nanoparticles are selected from the group consisting of plasmonic metal nanoparticles, plasmonic carbon nanotubes, plasmonic organic nanoparticles, plasmonic conductive oxides and plasmonic conductive transition metal nitrides, hybrids, silica coated nanoparticles or mixtures thereof, preferably plasmonic metal nanoparticles, wherein the metal is selected from the group consisting of Au, Al, Ag, Cu, Pt and Pd, most preferably Au.

5. Composition according to any one of claims 1 to 4, wherein the photothermal dyes are near-infrared absorbing dyes, preferably selected from the group consisting of indocyanine green, brilliant blue green, bromophenol blue, 4-[2-[2-chlor-3-[(2,6-diphenyl-4*H*-thiopyran-4-yliden)-ethyliden]-1-cyclohexen-1-yl]ethenyl]-2,6-diphenylthiopyrylium-tetrafluoroborat (IR-1061), diimminium dye and cyanine dyes.

6. Composition according to any one of claims 1 to 5, wherein comprising neodymium-doped BiVO₄ as fluorescent nanothermometers and TiN and/or gold nanorods as plasmonic nanoparticles.

7. Composition according to any one of claims 1 to 6, wherein the excitation maximum of the fluorescent nanothermometer differs from the absorption maximum of the plasmonic nanoparticle by at least 200 nm.

8. Composition according to any one of claims 1 to 7, **characterized in that** the temperature sensitive solder solder is selected from the group consisting of serum albumin, chitosan, collagen, fibrinogen and gelatin or a mixture thereof, preferably bovine serum albumin (BSA), human serum albumin (HAS) and gelatin or a mixture thereof, most preferably a mixture of bovine serum albumin (BSA) and gelatin or human serum albumin (HAS) and gelatin.

9. Composition according to claim 8, wherein the temperature sensitive solder additionally comprises a biodegradable polymer selected from the group consisting of poly(L-lactic acid) (PLA), poly(glycolic acid), poly(L-lactic-co-glycolic acid) (PGA), poly([epsilon]-caprolactone) (PLGA) and polyortho esters, polyanhydrides or combinations thereof.

10. Composition according to any one of claims 2 to 9, **characterized in that** the fluorescent nanothermometers and the plasmonic nanoparticles are present in a ratio of 1:1 to 5000:1 % by weight.

11. Composition according to any one of claims 1 to 10, wherein the composition comprises 0.01 to 100 mg/ml, preferably 0.1 to 50 mg/ml, most preferably 1 to 10 mg/ml of the fluorescent nanothermometers.

12. Composition according to any one of claims 1 to 11, wherein the composition additionally comprises a therapeutic agent selected from the group consisting of antimicrobial agents, anti-inflammatory agents and angiogenic agents.

13. Composition according to any one of claims 1 to claim 12, wherein the composition is formulated as paste, gel, strip, patch or viscous fluid having a viscosity of at least 2'000 centipoises at 25°C.

14. Composition according to any one of claims 1 to 12, wherein the composition is in lyophilised form.

15. Composition according to any one of claims 1 to 14 for use in the treatment of biological tissue repair, wherein the tissue is preferably selected from the group consisting of cornea, blood vessels, fascia, diaphragm, anastomosis of vessels and tissues, dura mater leaks, gastrointestinal tissues and nerves.
